# EUROPEAN PATENT APPLICATION

(11) **EP 0 788 777 A1**
(43) Date of publication of application: **13.08.1997**
(21) Application number: 96610004.2
(22) Date of filing: 06.02.1996
(51) Int. Cl.: A61B 19/08

(54) **Covering for protection of a medical instrument**

(71) Applicant: NIKOMED APS, DK-2610 Roedovre (DK)
(72) Inventor: Kornerup, Niels, DK-2610 Rodovre (DK)
(74) Representative: Nielsen, Henrik Sten

(57) **Abstract**

An elongated protective covering for protection of an instrument for medical use comprises an elongated, flexible tube defining a proximal and a distal end. The proximal end presents an insertion aperture, and the distal end presents an exit section provided with an aperture for the exsertion of the instrument. The protective covering also comprises a foldable, reinforcing plate connected to and reinforcing the elongated, flexible tube at the proximal end and defining two states: a first, collapsed, plane state, wherein the insertion aperture is collapsed, and a second, expanded state, wherein the insertion aperture is open; and a resilient force generating means connected with the foldable, reinforcing plate and acting in conjunction therewith for shifting the foldable, reinforcing plate from the first state to the second state and maintaining the foldable, reinforcing plate in the second state when not exposed to external forces, thereby allowing the instrument to be introduced into the elongated, flexible tube through the insertion aperture.

## Description

The present invention relates to an elongated protective covering for protection of an instrument for medical use, and a unitary package containing an elongated protective covering.

In the medical field and, in particular in operation rooms, it is frequently necessary during medical examinations or during surgery to employ surgical or optical instruments as endoscopes, arthroscopes or surgical lasers which have to be protected from septical particles. As these medical instruments often present considerable dimensions and therefore are difficult to sterilize, an elongated, flexible, sterile, protective cover is provided for their antiseptic protection.

Such covers are known from US-A-3 809 072 and DE-C-0 406 507, to which reference is made and which are hereby incorporated in the present specification by reference.

Another type of elongated, flexible, sterile, protective cover is disclosed in EP 0 371 909 B1 and comprises an elongated, flexible tube with retractable folds allowing that the protective cover may be stored in a compacted state and be unfolded through unfolding the retractable folds. The protective cover defines a proximal end and a distal end, and the proximal end and the distal end represent an insertion aperture and an exit aperture, respectively. The insertion aperture is provided with two flexible, lamellar linings having opposite, substantially congruent edges. The linings define six outwardly oriented, tubular, segment-like curvatures, the axes of which extend in the direction of the longitudinal extent of the tubular sheeting, enabling the linings to be spread open like jaws by pressing onto their edges in order to open the insertion port, thereby producing an aperture of a hexagonal configuration.

Considering that the opening of the insertion port and the introduction of the medical instrument in the protective cover is carried out by a person who already holds the instrument in his/her hand, the configuration has three main disadvantages; firstly, the insertion aperture can only be opened by manual power by pressing the linings onto their edges; secondly, the pressing of the linings is limited by the hand/palm span, i.e. a breadth of maximum 20 cm, with the result that the use of broader protective covers is prevented, being conditioned by the use of both hands, and, thirdly, the expanded insertion aperture configuration is not stable, being insufficiently stiff and, therefore, susceptible to collapse after having been pressed onto the edges to expand.

An object of the present invention is to provide an elongated disposable protective covering for protection of an instrument for medical use which eliminates the above-mentioned disadvantages of the prior art protective covers.

A particular object of the present invention is to provide a unitary, sterilized, disposable protective covering which is not limited to a specific breadth corresponding to the average size of the hand of a nurse or surgeon.

An advantage of the present invention is that the unitary, sterilized, disposable protective covering according to the present invention may be produced in any arbitrary size, including any arbitrary width for accommodating and receiving an instrument of a specific size, still providing an improved function and easier opening of the insertion aperture as compared to the prior art protective coverings.

A particular feature of the present invention is that the unitary, sterilized, disposable protective covering according to the present invention provides an automatic opening of the insertion aperture after the protective covering has been removed from an enclosing, sealed package and is kept in a state in which the insertion aperture is open, allowing the easy introduction of the instrument into the protective covering and also an easy removal of the instrument from the protective covering, still allowing that the insertion aperture may be closed by simply pressing the insertion aperture for collapsing the insertion aperture or the lining thereof and optionally sealing the insertion aperture, e.g. by means of a piece of adhesive tape or the like.

The above objects, the above advantage, and the above feature, together with numerous other objects, advantages, and features which will be evident from the below detailed description of preferred embodiments of the present invention are in accordance with the teachings of the present invention obtained by an elongated protective covering for protection of an instrument for medical use, comprising: an elongated, flexible tube means defining a proximal and a distal end, said proximal end presenting an insertion aperture and said distal end presenting an exit section provided with an aperture for the exsertion of said instrument,
a foldable, reinforcing plate means connected to and reinforcing said elongated, flexible tube means at said proximal end and defining two states: a first, collapsed, plane state, wherein said insertion aperture is collapsed, and a second, expanded state, wherein said insertion aperture is open; and
a resilient force generating means connected with said foldable, reinforcing plate means and acting in conjunction therewith for shifting said foldable, reinforcing plate means from said first state to said second state and maintaining said foldable, reinforcing plate means in said second state when not exposed to external forces, thereby allowing said instrument to be introduced into said elongated, flexible tube means through said insertion aperture.

Due to the provision of resilient force generating means connected with the foldable reinforcing plate means, the insertion aperture of the elongated flexible tube means of the protective covering is easily opened, as the foldable, reinforcing plate means is simply shifted to the second state, causing the insertion aperture to be opened as the protective covering is removed from an enclosing sealed package in which the elongated protective covering is kept in a sterilized state prior to use. Through the provision of the resilient force generating means characteristic of the present invention, the size of the foldable reinforcing plate means and also the width of the elongated flexible tube means of the protective covering according to the present invention may be configurated in any appropriate size, irrespective of the maximum hand/palm span of the person who is to use the protective covering.

The elements or means of the elongated protective covering according to the present invention may be configurated or implemented in accordance with any specific requirements fulfilling any specific purposes, as the individual elements or means of the elongated protective covering according to the present invention may be produced from elements well-known in the art per se, comprising plastics and cardboard materials, including plastic tubes or hoses and cardboard or plastic foils or sheets, etc. Alternatively, or additionally, metal foils such as aluminium foils may for certain applications be used together with or as an alternative to the plastic elements, such as the plastic hose or tube and/or plastic plates constituting the reinforcing plate means.

According to presently preferred embodiments of the elongated protective covering according to the present invention, the reinforcing plate means is made from a fairly stiff material, such as cardboard or plastic or metal film. The expression a fairly stiff material is to be construed defining a material, which exhibits a stiffness which as compared to the elongated flexible tube means provides the intentional reinforcing of the elongated flexible tube means for fulfilling the purpose of providing, in conjunction with the elongated flexible tube means, the collapsible and expandable reinforcing plate means allowing that the insertion aperture is closed in the above first state and open in the above second, expanded state of the foldable, reinforcing plate means.

The foldable, reinforcing plate means which defines the insertion aperture as the foldable, reinforcing plate means is in the above second, expanded state may produce an insertion aperture of any specific configuration, such as a triangular, square, quadratic, pentagonal, hexagonal, or generally polygonal configuration. The configuration of the insertion aperture defined by the foldable reinforcing plate means in the above second expanded state may also optionally include plane or curved segments or combinations thereof, e.g. include plane plate segments exclusively, curved plate segments exclusively, or a combination of plane and curved plate segments.

The elongated flexible tube means of the protective covering according to the present invention basically have to fulfil the main intentional purpose of covering the instrument as the protective covering is used for enclosing and encapsulating the instrument within the elongated flexible tube. For adapting the specific configuration of the instrument, and also allowing the instrument to be introduced into the tube means and later on removed from the tube means, the tube means has to be a flexible tube means which further preferably allows that the flexible means may be easily folded or otherwise compacted prior to use for reducing the overall size of the protective covering as the protective covering is stored prior to use, preferably in a sealed, evacuated package in which the protective covering is kept in a sterilized state. For allowing that the elongated flexible tube means may be compacted and kept in a compacted state prior to use, the elongated flexible tube means may have retractable, telescopic folds which superpose each other starting with the distal end, or alternatively have retractable telescopic folds which superpose each other, starting with the proximal end.

In alternative embodiments of the elongated protective covering according to the present invention allowing that the elongated flexible tube means may be compacted and kept in a compacted state prior to use, the flexible tube means is foldable in an accordion-like fashion or may be rolled into a toroid configuration and is kept in a toroid configuration as the rolling is started at the distal end, or alternatively started at the proximal end.

The materials used for the elongated protective covering according to the present invention comprise, as stated above, plastic materials, and the flexible tube means is in accordance with preferred and alternative embodiments made from polyethylene, polypropylene, PVC or the like, or a combination of the above or any plastics material well-known in the art per se.

In accordance with specific requirements as to the exit section presented at the distal end of the elongated flexible tube means, the distal end may be configurated in any specific configuration, e.g. an inwardly and outwardly tapering configuration, a polygonal configuration or a configuration defined by straight-line or curved-line boundaries, including hemispherical or oval configurations. The exit section of the elongated flexible tube means may be made from the same material as the elongated flexible tube means, or alternatively be made from a different material fulfilling any specific requirements as to flexibility and/or stiffness, or alternatively optic transparency or radiofrequency transparency or radiofrequency intransparency. The elongated flexible tube means may be made from a light-transparent or a non light-transparent material, and similarly, the exit section of the elongated flexible tube means may be made from a transparent material or a non-transparent material, such as an opaque material.

The exit section of the elongated flexible tube means of the protective covering according to the present invention may constitute a sealed section which is perforated prior to use, e.g. by means of a knife or a pair of scissors, or alternatively have a sealing foil, such as a flexible rubber or plastic foil which is perforated prior to use for providing a hermetical seal relative to a component protruding from the instrument which is contained and sealed within the protective covering according to the present invention. According to the presently preferred embodiment of the protective covering according to the present invention, the exit section is provided with at least one precut circular or polygonal, or alternatively oval aperture through which an elongated element such as an optic fiber of the instrument may protrude.

The resilient force generating means of the protective covering according to the present invention may in accordance with alternative embodiments be configurated in numerous ways obvious to a person having ordinary skill in the art, as the resilience of the resilient force generating means may easily be produced by any appropriate resilient means, such as springs, resilient materials, e.g. rubber or plastic materials, etc. Thus, in accordance with alternative embodiments of the protective covering according to the present invention, the resilient force generating means is produced by springs, resilient rubber or plastic strings, belts, plates, etc. which are provided for producing the shifting of the foldable reinforcing plate means from the first collapsed plane state to the second expanded state and maintain the foldable, reinforcing plate means in the second state when not exposed to external forces, i.e. after the protective covering is removed from the enclosing package in which the protective covering is kept in a sterilized state prior to use.

In accordance with a first embodiment of the protective covering according to the present invention, the resilient force generating means is simply produced by resilient plate segments which constitute hinges interconnecting plate elements of the foldable reinforcing plate means. According to an alternative and presently preferred embodiment of the elongated protective covering according to the present invention, the resilient force generating means includes an elastic string, such as a rubber band which passes through holes or cut-outs provided in the reinforcing plate means around its perimeter, which elastic string has a length slightly shorter than the perimeter of the reinforcing plate means.

According to a further alternative embodiment of the protective covering according to the present invention, the resilient force generating means includes a pair of scissors-like extenders connected to the extremities of the foldable, reinforcing plate means and provided with an elastic string, such as a rubber band or rubber belt biasing the reinforcing plate means towards the second state.

The connection between the foldable reinforcing plate means and the elongated flexible tube means may be accomplished in any specific and appropriate manner as the foldable reinforcing plate means may be connected to the exterior side or the interior side of the flexible tube means, dependent on the actual intentional use of the elongated protective covering and the specific configuration of the flexible tube means and/or the foldable reinforcing plate means. According to a further alternative, the foldable reinforcing plate means is enclosed within a turned-in part of the flexible tube means, in which embodiment the resilient force generating means is preferably constituted by the above described elastic string passing through holes or cut-outs provided in the reinforcing plate means which is consequently also enclosed within the turned-in part of the flexible tube means providing an overall encapsulated structure in which the foldable reinforcing plate means and the resilient force generating means is concealed within an enclosed and sealed compartment defined by the flexible tube means.

The present invention also relates to a unitary package containing an elongated protective covering for protection of an instrument for medical use.

The above objects, the above advantages and the above features together with numerous other objects, advantages and features which will be evident from the below detailed description of preferred embodiments of the present invention are in accordance with the teachings of the present invention obtained by a
unitary package according to the present invention comprising: a sealed evacuated package, and an elongated protective covering contained within said package and comprising:
an elongated, flexible tube means defining a proximal and a distal end, said proximal end presenting an insertion aperture and said distal end presenting an exit section provided with an aperture for the exsertion of said instrument,
a foldable, reinforcing plate means connected to and reinforcing said elongated, flexible tube means at said proximal end and defining two states: a first, collapsed, plane state, wherein said insertion aperture is collapsed, and a second, expanded state, wherein said insertion aperture is open; and
a resilient force generating means connected with said foldable, reinforcing plate means and acting in conjunction therewith for shifting said foldable, reinforcing plate means from said first state to said second state and maintaining said foldable, reinforcing plate means in said second state when not exposed to external forces, thereby allowing said instrument to be introduced into said elongated, flexible tube means through said insertion aperture.

The elongated protective covering of the unitary package according to the present invention may in accordance with the teachings of the present invention have any of the characteristics of the elongated protective covering according to the present invention as described above.

The present invention will now be further described with reference to the drawings, in which
Fig. 1 is a perspective and schematic view of a first embodiment of a unitary, sterilized, disposable, protective covering according to the present invention, contained in a sealed, disposable, sterilized package;
Fig. 2 is a perspective and schematic view of the first embodiment of the unitary, sterilized, disposable, protective covering according to the present invention, partly unfolded and with an insertion aperture partly expanded;
Fig. 3 is a perspective and schematic view similar to the view of Fig. 2 illustrating the use of the protective covering according to the present invention and also illustrating an instrument for the medical use partly received within the covering;
Fig. 4 is a sectional, longitudinal view of the first embodiment of the protective covering shown in figs. 2 and 3 partly unfolded and with the insertion aperture in a collapsed state;
Fig. 5 is a top view of the first embodiment of the protective covering shown in figs. 2, 3 and 4, with the insertion aperture in the collapsed state;
Fig. 6 is a perspective and schematic view of the insertion aperture of the first embodiment shown in figs. 2, 3, 4 and 5 in an expanded state; and
Fig. 7 is a perspective and schematic view of an insertion aperture of a second embodiment of the protective covering according to the present invention in the expanded state.

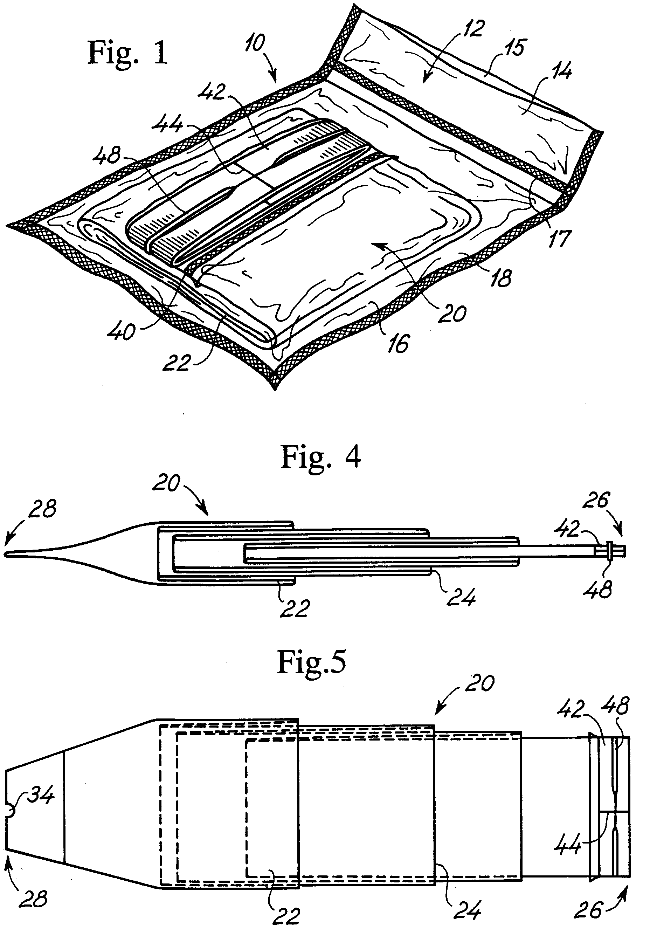

In Fig. 1, a first embodiment of a unitary, sterilized, disposable protective covering according to the present invention, contained within a sealed package is shown, designated by the reference numeral 10 in its entirety. The unitary, sterilized, disposable protective covering is designated the reference numeral 20 and is contained in a sealed, disposable, sterilized package 12. The package 12 is composed of two impermeable plastic foils 14 and 15 which are welded together along a peripheral edge, or otherwise joined together, e.g. by means of an adhesive as indicated by the reference numeral 16. The plastic foils 14 and 15 are further joined together along a transversal weld seam 17 and define an inner evacuated chamber 18 in which the disposable protective covering 20 is contained and sealed in a sterilized state. The protective covering 20 is kept in the sterilized package 12 during transportation ad storage as the sterilized package 12 is not opened until the covering 20 is to be used in a hospital or similar location, e.g. for surgery or other medical purpose.

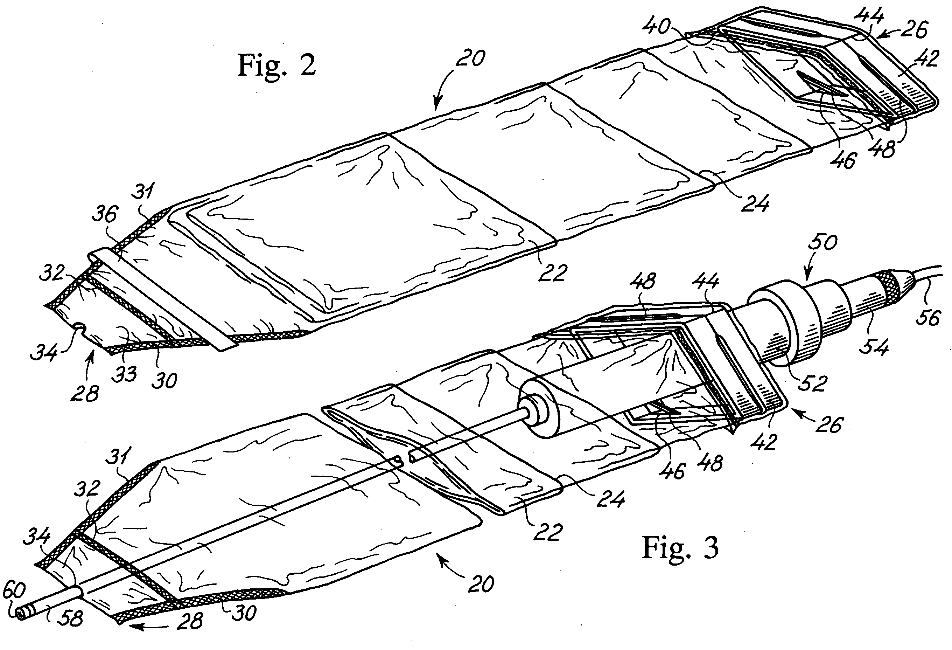

In Fig. 2, the protective covering 20 is shown in greater details, partly unfolded, disclosing the structure of the protective covering and also a particular feature relating to a self-opening ability of the protective covering as will be discussed in greater details below. The protective covering 20 basically comprises an elongated flexible tube 22 made from e.g. a plastic foil and defining opposite proximal and distal ends 26 and 28, respectively. The flexible tube 22 is for reducing the overall size of the protective covering 20, as the protective covering is stored within the sterilized package 10, described above with reference to Fig. 1, folded as the flexible tube 22 is arranged in folded overlapping relation as indicated at 24. The tube 22 may, of course, be compacted in a different way, e.g. by simply arranging the tube 22 in layers on top of one another.

At the distal end 28, the flexible tube 22 is provided with a narrowing defined by two welded joints 30 and 31 which define a tapering end or narrowing end of the tube. At the distal end, a separate foil end 33 is welded to the elongated tube 22 along a transversal welded joint 32 and defines an aperture 34 through which an elongated instrument, such as an optical fiber, as is illustrated in Fig. 3, and as will be described in greater details below, may extend. An adhesive tape 36 is further provided at the distal end 38 of the tube 22 by means of which the flexible tube 32 may be sealed round the instrument received within the elongated tube by simply adhering the adhesive tape 36 to the wall of the flexible tube 22 after the removal a release paper and at the same time pressing the wall of the tube round the instrument.

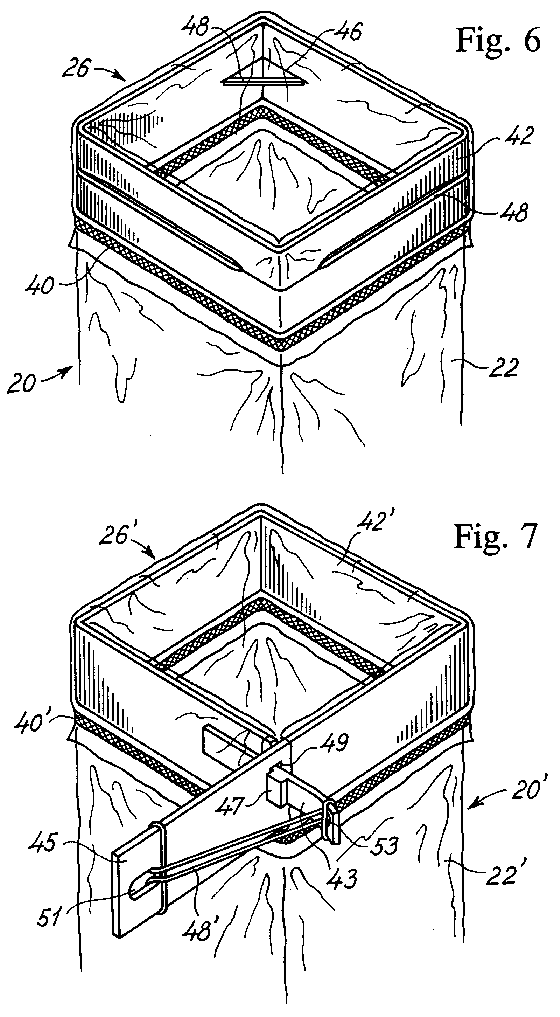

At the proximal end 26, a casing or passage is defined as the wall of the tube 22 is folded onto itself and welded along a circumferential welded joint 40. Within the passage or casing, a circumferential band made from cardboard is received. The band is designated the reference numeral 42 and is divided into a total of four cardboard plate elements as the cardboard band is weakened along weakening lines, one of which is designated the reference numeral 44, providing hinges between two adjacent plate elements of the band. As is evident from Fig. 4, a total of four plate elements of the band 42 are provided, however, the band 42 may along weakening lines defining hinge connections be divided into any arbitrary number, e.g. six, eight, or even more plate segments. The plate segments are in a first state collapsed into a compacted configuration in which two of the plate segments are arranged on top of the two remaining plate segments defining a substantially plane overall configuration of the band 42. The band 42 may be raised to a second state in which a basically rectangular aperture is defined by the band 42. The individual plate elements of the band 42 are preferably of different width for allowing the band to be easily raised to the above second state from the above first or collapsed state by means of a elastic rubber band which is designated the reference numeral 48 and extends circumferentially round the belt 42 as the elastic rubber band 46 is received in cut-outs 48 which extend perpendicularly to two opposite hinges 44. In the above first or collapsed state, the elastic rubber band 46 is slightly stretched as compared to the above second state in which the belt 42 is raised into a configuration in which a substantially rectangular aperture is defined by the raised belt 42. Provided the individual plate elements of the bands 42 were of identical size, the elastic rubber band 48 might not be able to cause a raising of the belt from the collapsed first state to the raised second state as the perfect symmetrical configuration would not give origin to any force imbalance causing the automatic raising of the belt 42 from the collapsed first state to the raised second state.

It is to be realized that the provision of the elastic rubber band 48 provides a means for automatic raising the belt 42 from the collapsed state to the raised second state in which an aperture is defined at the distal end 46 allowing a person to introduce an instrument, such as an instrument 50 shown in Fig. 3 into the interior of the covering 20 through the aperture defined by the raising of the belt 42. Furthermore, contrary to prior art elastic coverings of the present type, the provision of the elastic rubber band 48 renders it possible to produce the covering in any arbitrary size and also a fairly large width allowing the covering to be used in connection with fairly large instruments.

In Fig. 3, the covering 20 is shown in a state in which the instrument 50 is being introduced into the interior of the covering, as the belt 42 is raised to a state in which a rectangular aperture is defined at the proximal end 26 of the covering 20. The instrument 50 comprises a housing 52 defining opposite first and second ends. At the first end, the housing 52 is connected to an electric cable 56 through an electric plug 54 and at the opposite second end, the housing 50 is connected to an optical fiber 58 which defines a distal end 60 at which the optic fiber is exposed. It is to be understood that the instrument 50 shown in Fig. 3 constitutes a single among a plurality of instruments which may be used in connection with the protective covering 20 according to the present invention.

In Fig. 4, the protective covering 20 is shown in a cross-sectional view illustrating the folding of the tube 22 in the above described overlapping relation along the folds 24 defining a compacted structure in which the covering 20 is contained within the package 10 described above with reference to Fig. 1.

In Fig. 5, a top view of the protective covering 20 is shown, illustrating the above described belt 42 and also the folding of the elongated tube 22 in the above described overlapping relation.

Fig. 6 discloses the proximal end 26 of the protective covering 20 in greater details, illustrating the circumferential band 42, and also the position of the rubber band 48 which extends through two opposite cut-outs 46 at two of the corners defined by the hinges described above. It is to be realized that different elastic or resilient elements, such as springs or flexible plastic materials having a memory and being of the recoverable type may be used for producing the advantageous feature characteristic of the protective covering according to the present invention, namely the automatic raising of the collapsed proximal end to a raised state after the protective covering is removed from the sterilized package in which the protective covering is confined and kept prior to use.

In Fig. 7, an alternative embodiment of the protective covering is shown designated the reference numeral 20'. In Fig. 7, elements or components fulfilling the same purpose as similar components or elements of the above described first embodiment 20 are designated the same reference numerals as the corresponding components or elements of the embodiment 20, however, added the additional sign '. The embodiment of the protective covering 20', shown in Fig. 7, basically differs from the above described first embodiment in that the belt 42 is substituted by a belt 42' which is provided with extenders 43 and 45 extending beyond a corner defined by the belt or frame 42'. The extender 43 is received within an aperture 49 defined in the extender 45 and is provided with an abutment 47 preventing the extenders 43 and 45 from being disconnected from one another. The extender 43 and the extender 45 are provided with through-going holes 53 and 51, respectively, in which a rubber band 48' is received, constituting a resilient element producing the self-raising capability of the frame 42' as the rubber band 48' is stretched as the frame 42' is collapsed and therefore produces a force through the stretching which urges the extenders 43 and 45 towards the position shown in Fig. 7 in which the frame 42' is kept in a raised position in which a substantially square aperture is defined at the proximal end 26' of the protective covering 22'.

### Example 1

A prototype implementation of the above described first and presently preferred embodiment of the protective covering according to the present invention described above with reference to Figs. 1-6 was made from the following components: The flexible tube 20 was a 2.5 m PP (polypropylene) tube defining a length of 2.5 m and a perimeter of 35 cm. The distal end 28 of the tube 22 was configurated as illustrated in Fig. 2 and the adhesive tape 36 was constituted by a conventional adhesive tape of a width of 13 mm and a length of approximately 12 cm having a protective covering applied to the one side constituting the side facing the wall of the tube 22. The belt 42 was made from a cardboard material defining a total perimeter of 35.5 cm and a width of 3.5 cm. Two of the plate elements of the band were measuring 9.0 cm x 3.5 cm, whereas the two remaining opposite plate elements were measuring 8.5 cm x 3.5 cm. The cut-outs 46 were produced in a symmetrical configuration relative to the adjacent hinges and the rubber band 48 was a conventional highly elastic natural rubber band of a perimeter in relaxed state of approximately 12 cm.

## Claims

1. An elongated protective covering for protection of an instrument for medical use, comprising:
an elongated, flexible tube means defining a proximal and a distal end, said proximal end presenting an insertion aperture and said distal end presenting an exit section provided with an aperture for the exsertion of said instrument,
a foldable, reinforcing plate means connected to and reinforcing said elongated, flexible tube means at said proximal end and defining two states: a first, collapsed, plane state, wherein said insertion aperture is collapsed, and a second, expanded state, wherein said insertion aperture is open; and
a resilient force generating means connected with said foldable, reinforcing plate means and acting in conjunction therewith for shifting said foldable, reinforcing plate means from said first state to said second state and maintaining said foldable, reinforcing plate means in said second state when not exposed to external forces, thereby allowing said instrument to be introduced into said elongated, flexible tube means through said insertion aperture.

2. The elongated protective covering tube means according to claim 1, said reinforcing plate means being made of a stiff material, as cardboard or plastic or metal film.

3. The elongated protective covering according to any one of the preceding claims, said insertion aperture at the proximal end having in said second, expanded state a polygonal or an arcuate configuration or a combination thereof.

4. The elongated protective covering according to any one of the preceding claims, said elongated, flexible tube means having retractable, telescopic folds which superpose each other starting with said distal end.

5. The elongated protective covering according to any of claims 1-3, said elongated, flexible tube means having retractable, telescopic folds which superpose each other starting with said proximal end.

6. The elongated protective covering according to any of claims 1-3, said elongated, flexible tube means being foldable in an accordion-like fashion.

7. The elongated protective covering according to any of claims 1-3, said elongated, flexible tube being rolled starting at said distal end.

8. The elongated protective covering according to any of claims 1-3, said elongated, flexible tube being rolled starting at said proximal end.

9. The elongated protective covering according to any one of the preceding claims, said flexible tube means being made of polyethylene, polypropylene, PVC or the like or a combination thereof.

10. The elongated protective covering according to any one of the preceding claims, said distal end of said elongated, flexible tube means providing an exit section of a polygonal or hemispherical or oval configuration.

11. The elongated protective covering according to claim 10, said exit section being formed from a flexible, opaque or transparent material different from or identical with the material of said flexible tube means.

12. The elongated protective covering according to claim 10 or 11, said exit section being provided with at least one substantially circular or polygonal or oval aperture.

13. The elongated protective covering according to any one of the preceding claims, said resilient force generating means including an elastic string which passes through holes or cut-ours provided in said reinforcing plate means around its perimeter, and said elastic string having a length slightly shorter than said perimeter.

14. The elongated protective covering according to any one of claims 1-12, said resilient force generating means including a pair of scissors-like extenders connected to the extremities of said foldable, reinforcing plate means, and provided with an elastic string biasing said reinforcing plate means towards said second state.

15. The elongated protective covering according to any one of claims 1-14, said foldable, reinforcing plate means being connected to said flexible tube means on the exterior side thereof.

16. The elongated protective covering according to any one of claims 1-14, said foldable, reinforcing plate means being connected to said flexible tube means on the interior side thereof.

17. The elongated protective covering according to any one of claims 1-14, said foldable, reinforcing plate means being enclosed within a turned in part of said flexible tube means.

18. A unitary package comprising:
- a sealed evacuated package, and
- an elongated protective covering contained within said package and comprising:
an elongated, flexible tube means defining a proximal and a distal end, said proximal end presenting an insertion aperture and said distal end presenting an exit section provided with an aperture for the exsertion of said instrument,
a foldable, reinforcing plate means connected to and reinforcing said elongated, flexible tube means at said proximal end and defining two states: a first, collapsed, plane state, wherein said insertion aperture is collapsed, and a second, expanded state, wherein said insertion aperture is open; and
a resilient force generating means connected with said foldable, reinforcing plate means and acting in conjunction therewith for shifting said foldable, reinforcing plate means from said first state to said second state and maintaining said foldable, reinforcing plate means in said second state when not exposed to external forces, thereby allowing said instrument to be introduced into said elongated, flexible tube means through said insertion aperture.

19. The package according to Claim 18, said elongated protective covering having any of the characteristics of the elongated protective covering according to any of the claims 2-17.
